# EUROPEAN PATENT SPECIFICATION

(11) **EP 0 457 462 B1**
(45) Date of publication and mention of the grant of the patent: **16.02.1994**
(21) Application number: 91303953.3
(22) Date of filing: 01.05.1991
(51) Int. Cl.: C07C 323/44, C07C 319/20

(54) **Process for preparing guanidine derivatives**
Verfahren zur Herstellung von Guanidinderivaten
Procédé pour la préparation de dérivés de la guanidine

(30) Priority: 07.05.1990 JP 115791/90
(43) Date of publication of application: 21.11.1991
(73) Proprietor: MITSUI PETROCHEMICAL INDUSTRIES, LTD., Tokyo 100 (JP)
(72) Inventor: Ishitoku, Takeshi, c/o Mitsui Petrochemical Ind., Kuga-gun, Yamaguchi-ken (JP); Takahashi, Katsuya, c/o Mitsui Petrochemical Ind., Kuga-gun, Yamaguchi-ken (JP); Tan, Hiroaki, c/o Mitsui Petrochemical Ind., Kuga-gun, Yamaguchi-ken (JP); Tomino, Ikuo, c/o Mitsui Petrochemical Ind., Kuga-gun, Yamaguchi-ken (JP); Kihara, Noriaki, c/o Mitsui Petrochemical Ind., Kuga-gun, Yamaguchi-ken (JP)
(74) Representative: Myerscough, Philip Boyd

(56) References cited:
- JP-A-60 092 257
- PATENT ABSTRACTS OF JAPAN, vol. 12, no. 491, (C-554)[3338], 21st December 1988; & JP-A-63 201 163 (MITSUI PETROCHEMICAL IND. LTD) 19-08-1988
- PATENT ABSTRACTS OF JAPAN, vol. 12, no. 496, (C-555)[3343] 23rd December 1988; & JP-A-63 208 566 (MITSUI PETROCHEMICAL IND. LTD) 30-08-1988
- PATENT ABSTRACTS OF JAPAN, vol. 12, no. 496, (C555)[3343] 23rd December 1988; & JP-A-63 208 565 (MITSUI PETROCHEMICAL IND. LTD) 30-08-1988
- Methoden der Organischen Chemie(Houben Weyl) Band E11 (1985) p.50

## Description

The present invention relates to a process for preparing a guanidine derivative of the formula (III): 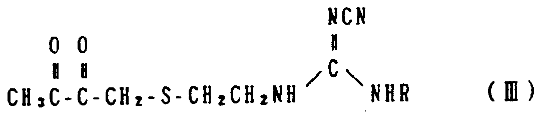
wherein R denotes lower alkyl, useful as an important intermediate for the synthesis of Cimetidine(N-cyano-N'-methyl-N''-[2-(S-methyl-1H-imidazol-4-yl)methylthioethyl]guanidine) and analogues thereof. Cimetidine has widely been used as a gastric acid secretion inhibitor based on the antagonistic action on histamine H₂-receptor.

The guanidine derivative (III) particularly N-cyano-N'-methyl-N''-[2-(2,3-dioxobutylthio)ethyl]guanidine, has been known to be produced by reacting cyanoguanidine derivative of the formula (IV) 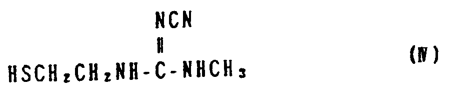
with a diacetyl derivative of the formula (II): 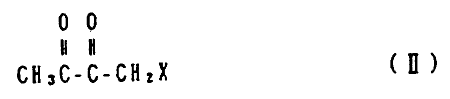
(Spanish Patent No. 455991, and Japanese Patent LOP-Publn. No. 92257/1985).

The method disclosed in the above Japanese Patent LOP-Publn. No. 92257/1985, however, involves the use of alkali metal hydride or an alcoholate at a temperature as low as -10°C, and both of the reagents are expensive and should be protected from contacting with water and therefore, they are difficult to handle. The method disclosed in the Spanish Patent does not produce a product of hight purity.

We have previously proposed a process comprising reacting a cyanoguanidine derivative (IV) with a diacetyl derivative(II) in the presence of an alkali metal -hydroxide or -carbonate (Japanese Patent LOP-Publn. No. 208565/1988), or in the presence of an organic base(Japanese Patent LOP-Publn. No. 208566/1988).

The cyanoguanidine derivative (IV) is an easily oxidizable liquid, has an unpleasant smell and is unstable during storage.

We have found that the above problems can be avoided by reacting a specific disulfide derivative with a specific diacetyl derivative under defined conditions.

Thus, the present invention provides a process for preparing a guanidine derivative of the formula (III): 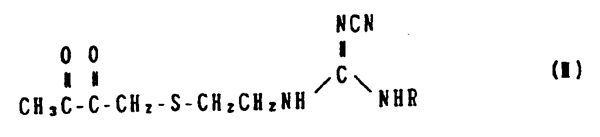
wherein R denotes lower alkyl, which comprises reacting a disulfide derivative of the formula (I): 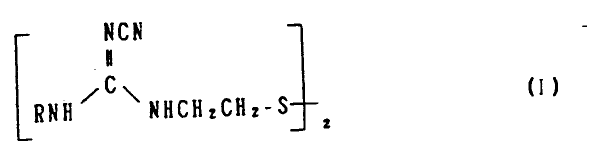
wherein R is as defined above, at a temperature of -30° to + 50°C with a diacetyl derivative of the formula (II) : 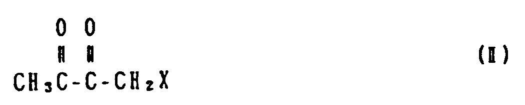
wherein X is halogen, in an amount of 1 to 5 moles per mole of said compound(I)in the presence of a thiol derivative in an amount of 0.5 to 5 moles per mole of said compound(I) and a basic compound in an amount of 0.3 to 3 moles per mole of said compound (I) and a solvent.

The disulfide derivative (I) used in the present invention has less smell, higher storage stability, easier to purify, and easier to handle than the cyanoguanidine derivative (IV).

The lower alkyl R in the formulas (I) and (III) may be straigth or branched, for example, methyl, ethyl, propyl, iso-propyl, n-butyl, sec-butyl or t-butyl. The halogen atom X in the diacetyl derivative (II) denotes chlorine or bromine, for example, chlorine being preferred.

The starting material disulfide derivative(I) used in the present process is known and can easily be prepared by known methods (for example, Japanese Patent LOP-Publn. No. 201163/1988). The disulfide derivative(I) prepared as above may be used directly without further purification, or used after purification by recrystallizing from a solvent such as methanol, ethanol, and the like.

The diacetyl derivative(II), also, is a known compound and may be prepared by halogenation of diacetyl.

Examples of the thiol derivative include alkylthiols such as methanethiol, ethanethiol, propanethiol, isopropanethiol, butanethiol, isobutanethiol, sec-butanethiol, t-butanethiol; alkylthiols the alkyl moiety of which is substituted by a group such as phenyl, amino, hydroxyl, alkoxyl, mercapto, alkoxycarbonyl etc., for example, benzylmercaptan, 2-aminoethanethiol, 2-mercaptoethanol, 2-methoxyethanethiol, ethanedithiol, propanedithiol, methyl thioglycolate; thiophenols such as thiophenol, 4-methylthiophenol.

The basic compounds which may be mentioned are inorganic bases such as sodium hydroxide, potassium hydroxide, potassium carbonate, sodium carbonate, sodium hydrogen carbonate; tertiary alkylamines such as triethylamine, tripropylamine; aromatic amines such as pyridine, aniline and N, N-dimethylaniline. Inorganic bases such as sodium hydroxide, potassium hydroxide are preferred.

The present process is carried out in the presence of a solvent. Suitable solvents are aliphatic alcohols such as methanol, ethanol, propanol, isopropanol, butanol, methyl cellosolve; ethers such as diethyl ether, tetrahydrofuran, dioxane, dimethoxyethane; nitriles such as acetonitrile, propionitrile; halogenated hydrocarbons such as dichloromethane, chloroform, carbon tetrachloride; amides such as formamide and dimethylformamide; and sulfoxides such as dimethyl sulfoxide, sulfolane. Preferably, methyl cellosolve, methanol, ethanol, or isopropanol is used.

In the practice of the present invention, the amount of the solvent used is in the range of 1-50 parts by weight, preferably 10-40 parts by weight based on one part by weight of the starting compound(I). The amount of the diacetyl derivative (II) is 1-5 mol, preferably 2-4 mol, per mol of the compound (I) . The amount of the thiol derivative is 0.5-5 mol, preferably 1-3 mol, per mol of the compound (I). The amount of the basic compound is 0.5-3 mol, preferably 0.7-1.5 mol, per mol of the compound (I).

The reaction of the present invention is carried out at -30°C to +50°C, preferably at 0°C to +30°C, for 10 minutes to 5 hours, preferably 30 minutes to 2 hours. After the reaction is complete, the reaction product can be isolated and purified according to conventional methods to give guanidine derivative of the formula(III).

Thus, the guanidine derivative(III) can be obtained by the present process using disulfide derivative(I) having less smell, capable of being easily purified and of higher storage stability than cyanoguanidine derivative(IV).

The following non-limiting Examples further illustrate the invention.

### Example 1

9.4 g(30 mmol) of cyanoguanidine compound(I) (R=Me) was dissolved in 300 ml of methyl cellosolve, and 10.8 g(90 mmol) of chlorodiacetyl was added to the solution. To the resultant mixture, 4.7 g(60 mmol) of 2-mercaptoethanol in 30 ml of an aqueous 2N sodium hydroxide was added and the mixture was stirred for 30 minutes at room temperature. An aliquot of the reaction mixture was analyzed by high performance liquid chromatography(column: ZORBAX-ODS, solvent: water/MeOH/AcOH/Et₃N 2000:1000:2.5:2.5), and found that the guanidine derivative(III) (R=Me) was formed in 25 % yield.

### Example 2

The procedure of Example 1 was followed using 7.5 g(60 mmol) of benzylmercaptane instead of 2-mercaptoethanol to give guanidine derivative(III) (R=Me) in 36 % yield.

### Example 3

The procedure of Example 1 was followed using 6.6 g(60 mmol) of thiophenol instead of 2-mercaptoethanol to give guanidine derivative(III) (R=Me) in 37 % yield.

### Example 4

The procedure of Example 1 was followed using 6.4 g(60 mmol) of methyl thioglycolate instead of 2-mercaptoethanol to give guanidine derivative(III) (R=Me) in 70 % yield.

## Claims

1. A process for preparing a guanidine derivative of the formula (III): 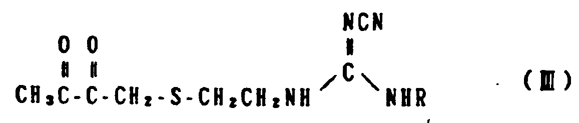 wherein R denotes lower alkyl, which comprises reacting a disulfide derivative of the formula (I): 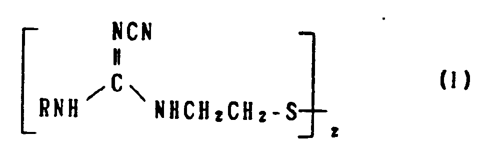 wherein R is as defined above, at a temperature of -30° to +50°C with a diacetyl derivative of the formula (II): 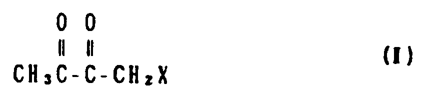 wherein X is halogen, in an amount of 1 to 5 moles per mole of said compound(I) in the presence of a thiol derivative in an amount of 0.5 to 5 moles per mole of said compound(I) and a basic compound in an amount of 0.3 to 3 moles per mole of said compound (I) and a solvent.

2. A process according to claim 1wherein the thiol derivative is an alkylthiol optionally substituted in the alkyl moiety by phenyl, amino, hydroxyl, alkoxyl, mercapto or alkoxycarbonyl or is a thiophenol.

3. A process according to claim 1 or 2 wherein the basic compound is an inorganic base, a tertiary alkylamine or an aromatic amine.

4. A process according to claim 1, 2 or 3 wherein the amount of (II) is 2 to 4 mols per mol of (I).

5. A process according to any one of the preceding claims wherein the amount of the thiol derivative is 1 to 3 mols per mol of (I).

6. A process according to any one of the preceding claims wherein the amount of the basic compound is 0.7 to 1.5 mols per mole of (I).

7. A process according to any one of the preceding claims wherein the reaction temperature is 0 to 30°C.

## Patentansprüche

1. Verfahren zur Herstellung von Guanidinderivaten der Formel (III): 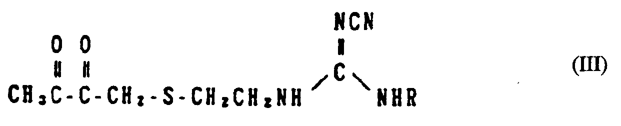 in der R für eine niedere Alkylgruppe steht, welches die Umsetzung eines Disulfidderivats der Formel (I): 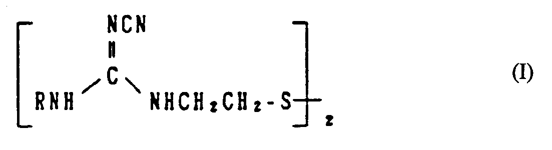 in der R wie oben definiert ist, bei einer Temperatur von -30°C bis +50°C mit einem Diacetylderivat der Formel (II) 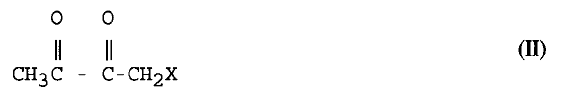 in der X ein Halogen bedeutet, in einer Menge von 1 bis 5 Mol pro Mol der Verbindung (I) in der Anwesenheit eines Thiolderivats in einer Menge von 0,5 bis 5 Mol pro Mol der Verbindung (I) und einer basischen Verbindung in einer Menge von 0,3 bis 3 Mol pro Mol der Verbindung (I) und einem Lösungsmittel umfaßt.

2. Verfahren gemäß Anspruch 1, wobei das Thiolderivat ein gegebenenfalls im Alkylteil durch Phenyl, Amino, Hydroxyl, Alkoxyl, Mercapto oder Alkoxycarbonyl substituiertes Alkyltiol oder Thiophenol ist.

3. Verfahren gemäß Anspruch 1 oder 2, wobei die basische Verbindung eine anorganische Base, ein tertiäres Alkylamin oder ein aromatisches Amin ist.

4. Verfahren gemäß Anspruch 1, 2 oder 3, wobei die Menge von (II) 2 bis 4 Mol pro Mol von (I) ist.

5. Verfahren gemäß mindestens einem der vorhergehenden Ansprüche, wobei die Menge des Thiolderivats 1 bis 3 Mol pro Mol von (I) ist.

6. Verfahren gemäß mindestens einem der vorhergehenden Ansprüche, wobei die Menge der basischen Verbindung 0,7 bis 1,5 Mol pro Mol von (I) beträgt.

7. Verfahren gemäß mindestens einem der vorhergehenden Ansprüche, wobei die Reaktionstemperatur 0 bis 30°C beträgt.

## Revendications

1. Procédé de préparation d'un dérivé de guanidine, de formule (III) : 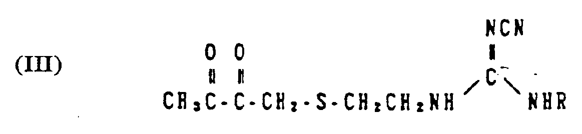 dans laquelle R représente un groupe alkyle inférieur,
qui comporte la réaction, à une température située entre -30°C et +50°C, d'un dérivé de type disulfure, de formule (I): 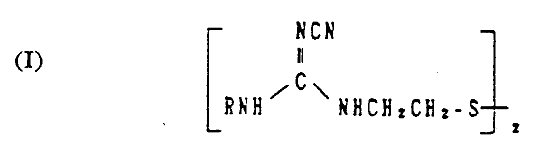 dans laquelle R a la même signification que ci-dessus,
avec de 1 à 5 moles, par mole dudit composé (I), d'un dérivé du diacétyle, de formule (II) : 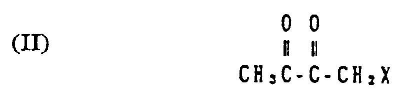 dans laquelle X représente un atome d'halogène,
en présence de 0,5 à 5 moles, par mole dudit composé (I), d'un composé de type thiol, de 0,3 à 3 moles, par mole dudit composé (I); d'un composé basique, et d'un solvant.

2. Procédé conforme à la revendication 1, dans lequel le composé de type thiol est un thiophénol, ou un alcanethiol dont le groupe alkyle porte éventuellement des substituants phényle, amino, hydroxy, alkoxy, mercapto ou alkoxycarbonyle.

3. Procédé conforme à la revendication 1 ou 2, dans lequel le composé basique est une base minérale, une alkylamine tertiaire ou une amine aromatique.

4. Procédé conforme à la revendication 1, 2 ou 3, dans lequel on met en jeu de 2 à 4 moles de composé (II) par mole de composé (I).

5. Procédé conforme à l'une des revendications précédentes, dans lequel on utilise de 1 à 3 moles du composé de type thiol par mole du composé (I).

6. Procédé conforme à l'une des revendications précédentes, dans lequel on utilise de 0,7 à 1,5 mole du composé basique par mole du composé (I).

7. Procédé conforme à l'une des revendications précédentes, dans lequel on effectue la réaction à une température située entre 0°C et 30°C.
